# EUROPEAN PATENT SPECIFICATION

(11) **EP 0 743 861 B1**
(45) Date of publication and mention of the grant of the patent: **16.04.2003**
(21) Application number: 95913955.1
(22) Date of filing: 23.01.1995
(51) Int. Cl.: A61K 51/00, A61K 38/00

(54) **RADIOLABELED ANNEXINS**
RADIOAKTIV-MARKIERTE ANNEXINE
ANNEXINES MARQUEES ISOTOPIQUEMENT

(30) Priority: 24.01.1994 US 185660
(43) Date of publication of application: 27.11.1996
(73) Proprietor: NEORX CORPORATION, Seattle Washington 98119 (US)
(72) Inventor: KASINA, Sudhakar, Kirkland, WA 98034 (US); DEWHURST, Timothy A., Seattle, WA 98119 (US); RENO, John M., Brier, WA 98036 (US); TAIT, Jonathan, Seattle, WA 98125 (US); STRATTON, John, Seattle, WA 98119 (US)
(74) Representative: Gowshall, Jonathan Vallance
(86) International application number: US9500953
(87) International publication number: WO95019791

(56) References cited:
- WO-A-91/07187
- WO-A-92/19279
- WO-A-95/34315
- WO-A-96/17613
- WO-A-96/17618
- US-A- 4 897 255
- US-A- 5 258 497
- US-A- 5 627 036
- KASINA S ET AL: "Preformed chelate Tc-99m radiolabeling of r-annexin V for arterial thrombus imaging" JOURNAL OF NUCLEAR MEDICINE, 37 (5 SUPPL.). 1996. 29P., XP002058829
- DEWHURST T.A. ET AL: "Tc-99m annexin V uptake by left atrial and coronary thrombi" JOURNAL OF NUCLEAR MEDICINE, vol. 35, no. 5, May 1994, pages 254p-255p, XP002091444
- STRATTON JR ET AL: "Selective uptake of radiolabeled annexin V on acute porcine left atrial thrombi." CIRCULATION, NOV 15 1995, 92 (10) P3113-21, XP002058828 UNITED STATES

## Description

### Related Application

This is a continuation-in-part of USSH 08/185,660 entitled Radiolabeled Annexins filed January 24, 1994 by Kasina et al.

### Technical Field

The present invention is directed to radiolabeled annexins. Also contemplated by the present invention are imaging protocols which involve the administration of a radiolabeled annexin. The annexin component of the conjugate serves to deliver the radiolabel active component of the conjugate to vascular thrombi target sites.

### Background of the Invention

When patients present with chest pain, palpitations or any other symptoms of coronary trauma or disease, the presence of vascular thrombi in the heart is a potential significant complicating factor for treatment. If a medical practitioner could non-invasively determine whether one or more vascular thrombi were present and, if present, the location of those vascular thrombi, better evaluation of treatment options would be possible. Furthermore, if a medical practitioner could determine that no vascular thrombi were present, thereby eliminating a potential complication in treatment, cardiac conditions could be treated more safely and effectively.

Most present techniques for determining the presence of vascular thrombi are invasive and/or cumbersome, and/or fail to detect such thrombi with good sensitivity and specificity. Thus, an imaging agent useful for non-invasive vascular thrombi imaging is desirable.

Annexins are a class of proteins that are characterized by calcium-mediated binding to anionic phospholipids. Anionic phospholipids are about 20 - fold more highly associated with activated platelets than quiescent platelets, and activated platelets are associated with vascular thrombi.

US-A-5627036 discloses detectably labelled annexines (sic) and compositions thereof.

Iodinated annexin V has been shown to localise to vascular thrombi in vivo, but has suboptimal imaging characteristics, possibly due to the pronounced beta phase of blood clearance owing to possible transiodination and/or metabolic degradation with reincorporation into serum macromolecules or non - target tissues. Free radioactive iodine or iodine - containing metabolic degradation products exposed non - target tissues, especially the thyroid gland, to radioactivity. In addition, the iodine radiolabel used is difficult to obtain and is not therefore practical for wide spread use. Consequently, improved radiolabeled annexin compounds are desirable.

### Summary of the Invention

The present invention provides radiolabeled annexins and methods of making and using the same. Preferred annexin - containing conjugates of the present invention are those suitable for radiolabeling with a diagnostic imaging agent including:
an annexin; and
an N₂S₂ or N₃S compound capable of complexing a radionuclide,
wherein the compound is covalently bound to the annexin.

A preferred annexin - containing conjugate is characterised by the following structure:

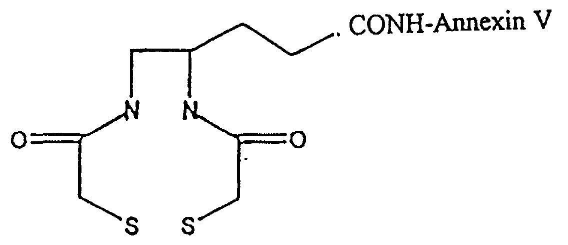

Also provided by the present invention are radiolabeled annexins suitable for imaging vascular thrombi, where radiolabled annexins include:
an annexin; or N₂S compound capable of complexing a radionuclide,
wherein the compound is covalently bound to the annexin;
a diagnostic radionuclide complexed by the chelate.

A preferred annexin for use in the present invention is annexin V. A preferred radiolabeled annexin for use in the present invention is characterised by the following structure:

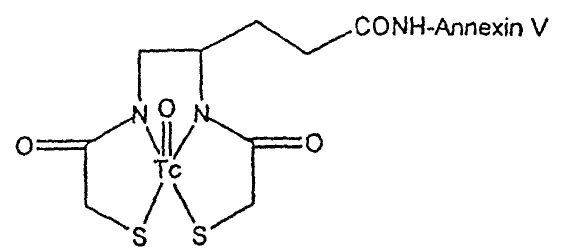

Preferred diagnostic radionuclides for use in the practice of the present invention are Tc-99m, Re-186 and Re-188, with Tc-99m being especially preferred. Vascular thrombi located in or near the heart are especially amenable to imaging in accordance with the present invention.

### Brief Description of the Drawings

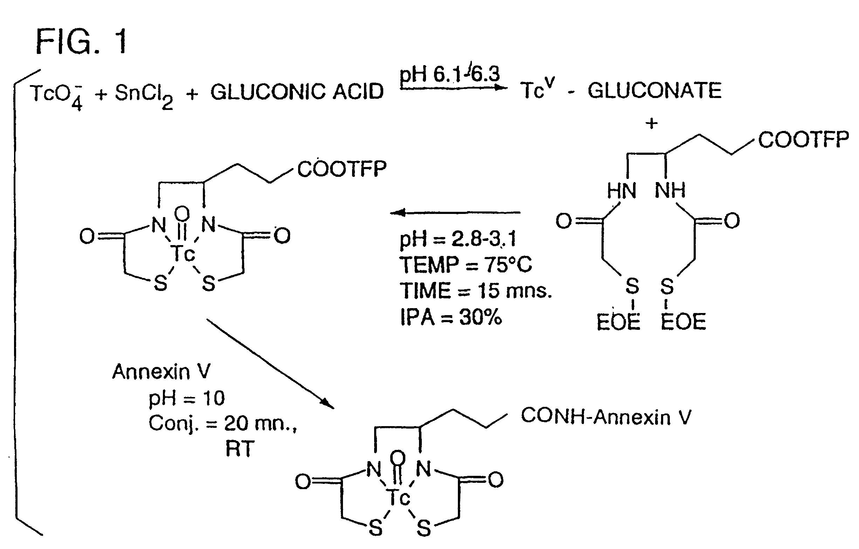

Fig. 1 schematically represents a method of radiolabeling annexin V.

Fig. 2 schematically represents a pET-12a plasmid Map.

Fig. 3 schematically represents pET-12a-PAP1, 3/7/94, clone 1.

### Detailed Description of the Invention

Prior to setting forth the invention, it may be helpful to set forth definitions of certain terms to be used within the disclosure.

Annexin: A class of compounds characterized by the ability to bind with high affinity to membrane lipids in the presence of millimolar concentrations of calcium. Annexins have been shown to exhibit anti-coagulatory effects that are mediated by the binding of annexins to negatively charged surface phospholipids (e.g., on activated platelets). This annexin-phospholipid binding is believed to block the activation of clotting factors by such negatively charged surface phospholipids. Prior to the recognition of the annexin class of molecules, members thereof were also referred to in the literature as placental anticoagulant proteins (e.g., PAP-1, 2, 3 and 4), lipocortins, calpactins, vascular coagulant (alpha and beta), calphobindin I, placental protein 4 (PP4), endonexin II, anchorin CII, calcium-dependent phospholipid binding protein, and the like. See Crumpton et al., Nature 345:212, 1990. Annexin-V is a prototypical annexin molecule used in the description of the present invention.

N₂S₂ Chelates: Diamide, dimercaptide bifunctional chelators of the NₓS_{y} family capable of stably complexing a radionuclide through two nitrogen atoms and two sulfur atoms that are appropriately positioned. Preferred N₂S₂ chelates are described in U.S. Patent No. 4,897,255, for example.

N₃S Chelates: Triamide, mercaptide bifunctional chelators of the NₓS_{y} family capable of stably complexing a radionuclide through three nitrogen atoms and one sulfur atom that are appropriately positioned. Preferred N₃S chelates are described in U.S. Patent No. 4,965,392, for example.

Conjugate: A conjugate encompasses chemical conjugates (covalently or non-covalently bound), fusion proteins and the like.

The present invention is directed to annexin-containing conjugates, radiolabeled annexins and the use thereof for diagnostic imaging purposes. The radiolabeled annexins of the present invention are characterized by the following: rapid accretion to target cell sites characterized by anionic phospholipids; short circulating half-life; in vivo stability against metabolic degradation or radionuclide separation from chelate; and amenability to packaging in cold kit format.

An embodiment of the present invention is directed to annexin-containing conjugates suitable for radiolabeling with a diagnostic imaging agent including:
an annexin; and
an N₂S₂ or N₃S compound capable of complexing a radio-nuclide, wherein the compound is covalently bound to the annexin. Radiolabeled annexins suitable for imaging vascular thrombi anywhere in the recipient (but particularly in or near the heart) are also contemplated, which radiolabeled annexins incorporate an annexin, an NₓS_{y} chelate, and further a diagnostic radionuclide complexed by the chelate.
Radiolabeled annexins suitable for imaging vascular thrombi are also contemplated, which radiolabeled annexins incorporate an annexin, an N₂S₂ chelate, and further a diagnostic radionuclide complexed by the chelate.

For the visualization of vascular thrombi associated with a number of pathological conditions, a conjugate of an annexin with a chelate complexed with an imaging radionuclide, such as Tc-99m for example, is administered to a recipient for whom such a diagnosis is desired. The annexin portion of the conjugate localizes rapidly to target sites characterized by negatively charged surface phospholipids, such as vascular thrombi. The radionuclide is selected for its ability to be visualized via one of the various techniques therefor, e.g., gamma camera imaging. Because of the rapid accretion of annexins to the target site and the short serum half-life (generally less than 30 minutes) of annexins (which is not significantly lengthened upon radiolabeling), imaging of those target sites proceeds with little exposure of non-target sites to radioactivity. Annexins are generally (with the most notable exception being annexin II) single chain, non-glycosylated proteins of approximately 33-72 kilodalton molecular weight. Annexins possess a number of biological associated with calcium ion-mediated binding.

Investigations have shown that annexins bind with high affinity to anionic membrane lipids in the presence of millimolar concentrations of calcium. In the presence of calcium, these proteins have an especially high affinity for negatively charged phospholipids, such as phosphatidylserine, phosphatidylglycerol, phosphatidic acid, or phosphatidylinositol. See, for example, Funakoshi et al., Biochem. 26: 5572-78, 1987; and Tait et al., Biochem. 27: 6268-76, 1988. Such negatively charged phospholipids are associated with vascular thrombi (e.g., are located on the surface of activated human platelets).

Annexins exert anti-coagulatory effects. Coagulation inhibition is mediated by the binding of annexins to negatively charged surface phospholipids (e.g., present on the surface of activated platelets). This binding is believed to block the activation of clotting factors by such negatively charged surface phospholipids. Annexins localize to target sites bearing anionic phospholipids rapidly, i.e., in a matter of approximately 5 to 30 minutes depending on circulating levels thereof, but remain circulating in the serum for a somewhat longer time period (circulating half-life < 30 minutes). Example III below discusses results of imaging experiments wherein vascular thrombi were visualized in planar images at an average time (following annexin administration) of 82 minutes.

Because of these properties, annexins or annexins conjugated to diagnostic or therapeutic agents may be employed in protocols for the *in vivo* diagnosis or treatment of vascular thrombi associated with a number of indications, such as DVT (deep vein thrombosis), PE (pulmonary embolism), myocardial infarction, atrial fibrillation, problems with prosthetic cardiovascular materials, stroke and the like. Other indications associated with accumulation of activated platelets, for which the annexin conjugates of the present invention are useful, include the following: abscess imaging, restenosis post balloon angioplasty (PCTA), inflammation of joints (*i.e*., Rheumatoid arthritis), damaged endothelial cells (*i.e.*, Alzheimer's disease), imaging of clots in cerebral arteries, occlusions in peripheral arteries, atrial thrombosis imaging, and imaging of coronary and carotid artery thrombi.

It is also important to characterize platelet populations in clinical, *in vitro* diagnostic, and basic research disciplines. Of the cell surface markers currently available to characterize platelets, many are not cross-reactive between species and may recognize all platelets as opposed to just the activated platelet population. It is believed that annexin selectively binds to activated platelets in many species. Thus, annexin conjugates of the invention can be utilized as an alternative cell marker in research and diagnostics disciplines such as immunohistochemistry and flow cytometry. For example, conjugates of the present invention can be used to detect activated platelets in fixed tissues/tumors, blood smears, in animals with coagulopathies, and *in situ* in platelet activation assays in response to various chemical or infectious stimuli as well as to detect activated platelets in blood, cell culture assays and platelet response assays. Exemplary diagnostic protocols and experimentation employing radiolabeled annexins are set forth below to further elucidate this aspect of the present invention.

An example of a preferred annexin useful in the practice of the present invention is Annexin V, which was isolated by Bohn in 1979 from human placenta, a rich source of annexins, and termed Placenta Protein 4 (PP4). Annexin V has been expressed in E. coli. Also, a full length cDNA clone of annexin V has been obtained and subcloned in expression vectors, thereby facilitating the production of fusion proteins containing annexin V. Annexin V consists of four domains (four tandem, imperfect repeats of about 75 amino acid residues, Funakoshi et al., Biochem. 26: 8087-92, 1987), wherein each domain is made up of 5 alpha helices. From the side, the annexin V molecule appears crown-like with at least four calcium binding sites on its convex surface, through which annexin-phospholipid interactions are mediated. Other annexin molecules are also useful in the practice of the present invention, and the discussions relating to annexin V herein apply generally to annexin molecules.

Because annexin V has a plurality of calcium binding sites, and because annexin V binding to negatively charged phospholipids is mediated by calcium, an engineered molecule consisting of one or more individual annexin V domains may be employed in imaging protocols of the present invention. Also, the annexin molecule may be partitioned at a position or positions different from the domain boundaries to provide an engineered molecule capable of calcium-mediated binding of anionic phospholipids. Also, annexin V may be altered at one or more amino acid residues, so long as the affinity of annexin V for anionic phospholipids is not significantly impaired. For example, the cysteine (position 316) amino acid residue of annexin V can be either deleted or replaced with alanine or other non-sulfur containing amino acid known to those skilled in the art; wherein, upon labeling, a monomerically radiolabeled annexin V is produced. The degree of annexin binding to phospholipids may be quantified by fluorescence quenching as described by Tait et al., J. Biol. Chem. 264: 7944-49, 1989).

Among annexins, annexin V has the strongest binding affinity (K_{d} < 10⁻¹⁰ M) for phospholipid vesicles containing 80% phosphatidylcholine and 20% phosphotidylserine under conditions comparable to plasma and extracellular fluid (1.2 mM ionized calcium, 0.15 M ionic strength). This binding is reversible and calcium dependent.

Annexin V is radiolabeled with an imaging radionuclide for use in the present invention. Radionuclides useful within the present invention include gamma-emitters, positron-emitters, Auger electron-emitters, X-ray emitters, fluorescence-emitters and the like. Radionuclides suitable for use in the present invention are known in the art and include ⁶⁴Cu, ¹⁸⁶Re, ¹⁸⁸Re, ¹⁰⁰Pd, ²¹²Bi, ²¹²Pb, ¹⁰⁹Pd, ⁶⁷Cu, ^{99m}Tc, ⁹⁴Tc, ⁹⁵Ru, ¹⁰⁵Ru, ⁹⁹Rh, ¹⁰⁵Rh, ¹¹¹In, ¹⁵³Sm, ¹⁷⁷Lu, ¹⁷⁰Lu, ¹⁸⁹Pt, ¹⁹³Pt, ¹⁹⁹Au, ¹⁹⁷Hg and the like.

Tc-99m is a preferred radionuclide for the practice of the present invention. Tc-99m has been stably bound to annexin V in accordance with the present invention at both low and high specific activity (0.53 µCi/µg - 101.2 µCi/µg). Adequate radiochemical yields and good radiochemical purities were obtained. Activated platelet binding studies were also conducted, and the radiolabeled annexin V conjugates bound to activated platelets well.

N₂S₂ and N₃S chelates are known in the art. For example, preferred N₂S₂ chelates are described in U.S. Patent No. 4,897,225, and preferred N₃S chelates are described in U.S. Patent No. 4,965,392. The present inventors have applied this stable chelation technology to exploit the thrombus-targeting ability of annexin molecules, thereby providing an imaging agent which is able to rapidly visualize vascular thrombi in vivo. The radiolabeled annexins of the present invention can be used to obtain thrombus images which reduce or eliminate the high level of background radioactivity that results from metabolically degraded radiolabeled conjugate. The radiolabeled annexins also avoid clinically unacceptable toxicity to non-target cell sites. Tc-99m radiolabeled annexin V performed better than I-123 in the pig studies set forth in Example III hereof.

Radiolabeling of annexin V with a radionuclide using an N₂S₂ or N₃S chelate may be conducted using either a pre-formed or a post-formed approach. That is, the radionuclide is either complexed within the chelate prior to (pre-formed) or following (post-formed) conjugation of the chelate to annexin V. The pre-formed approach is preferred and suitable procedures therefor are set forth in Examples I and II. Further studies indicated that the pre-formed radiolabeling approach yields two radiometric peaks by HPLC analysis. The appearance of two peaks results from cysteine-conjugated annexin V as well as lysine-conjugated annexin V. Thus, the cysteine amino acid of annexin V can be either deleted or replaced with other non-sulfur containing amino acid to result in a monomerically labeled annexin V, see Example V.

Annexin molecules may be modified by the addition of from about 2 to about 6 terminal amino acid residues to facilitate the conjugation reaction between the annexin molecule and the chelate. This modification may be made by protein chemistry methods or via production of an appropriate fusion protein or other techniques useful therefor.

Radiolabeled annexins of the present invention offer an additional advantage over the previously prepared I-123-labeled annexins, in that they are amenable to packaging in a cold kit. That is, the annexin and chelate components may be individually vialed and provided separately from the Tc-99m component (and, possibly, vialed separately from each other). When a patient requiring a thrombus image is identified, a cold kit may be ordered or retrieved from storage; Tc-99m may be obtained from a radiopharmacy or other source thereof; the pre-formed or post-formed chelation/complexation process is performed; the radiolabeled annexin is administered to the patient; and the patient is subsequently imaged.

Lyophilization and vialing in a sterile, pyrogen-free environment of the conjugate components may be accomplished via techniques, known to persons skilled in the art, of good manufacturing practices, particularly as such practices relate to biological materials.

Radiolabeled annexins of the present invention are administered in such amounts as to deliver a diagnostically effective amount of radionuclide to target sites. Appropriate administered doses depend on a variety of factors that are largely patient specific. The components of the radiolabeled annexin also impact dose amounts in ways that are known to or routinely ascertainable by practitioners in the art. In general, radiolabeled annexin is administered to large mammals at a dose ranging between about 0.3 and about 300 µg/kg body weight of the recipient, with from about 3 to about 10 µg/kg preferred, depending upon the physiological characteristics of the patient and the ailment involved or suspected. A practitioner in the art is capable of identifying an appropriate dose and administration route for a given recipient with a given ailment.

Radiolabeled annexins of the present invention may be administered in any convenient manner therefor. For example, intravenous infusion may be employed to administer radiolabeled annexins. Other routes of administration also find utility in the practice of the present invention. Exemplary additional administration routes are injection by the arterial (e.g., coronary artery), intracoronary, intralymphatic, intrathecal, or other intracavity routes, and the like.

After administration of the radionuclide, depending upon the nature of the radionuclide and the purpose of the administration, the recipient may be subject to various procedures for detection of radioactive emissions from the site or sites at which the radionuclide localizes. For example, conjugates containing Tc-99m are imageable by a gamma camera.

The invention is further described through presentation of the following examples. These examples are offered by way of illustration, and not by way of limitation.

### Example I

### Procedure for Radiolabeling an Annexin - N₂S₂ Chelate conjugate

Annexin V can be isolated from a variety of tissue extracts, such as liver, lung and placenta, in accordance with procedures set forth in Funakoshi et al., Biochem. 26: 8087-92, 1987); Tait et al., Biochem. 27: 6268-76, 1988; and U.S. Patent No. 4,937,324, for example. In addition, annexin V can be expressed in E. coli, as described by Tait et al., Archives of Biochemistry and Biophysics 288: 141-44, 1991.

Annexin V was radiolabeled with Tc-99m by using a diamide dimercaptide N₂S₂ chelate in accordance with the OncoTrac® Small Cell Lung Cancer Imaging Kit labeling procedure described in J. Nucl. Med. 32: 1445-51, 1991.

A preferred method for radiolabeling annexin V with Tc-99m constitutes a modified OncoTrac® kit procedure using C-18 Baker purified Tc-99m-N₂S₂-TFP. In this procedure, an acidified active ester solution was prepared by adding 0.16 ml of 0.2M hydrochloric acid: glacial acetic acid (14:2 ratio) to 0.6 ml of 2,3,5,6,-tetrafluorophenyl 4,5-bis-(S-1-ethoxy-ethylmercaptoacetamido)pentanoate (0.3 mg, 0.0005 mole freshly dissolved in 0.9 ml of isopropyl alcohol). Then 0.5 ml of this solution was added to 1.1 ml of Tc-99m-gluconate (prepared from 0.12 mg SnCl₂ 2 H₂O, 5.0 mg sodium gluconate at pH 6.1-6.3, and 100 mCi/ml of [Tc-99m] pertechnetate, i.e., the first step in the OncoTrac® kit labeling procedure). The reaction mixture was heated at 75°C for 15 minutes followed by cooling on ice. The resulting Tc-99m transchelated tetrafluorophenyl active ester derivative of Tc-99m-4,5-bis(thioacetamido)pentanoate was purified by loading the reaction mixture on a conditioned C-18 cartridge (J.T. Baker), washing with 2.0 ml water eight times followed by drying the column for 5 minutes, and eluting with 100% acetonitrile. The solvent was evaporated under a steady stream of N₂. Then 0.15 ml of phosphate buffered saline (PBS), 0.15 ml of annexin V at 2.35 mg/ml, and 0.2 ml of 0.2 M bicarbonate (pH 10.0) were added for conjugation to Tc-99m-N₂S₂. After 20 minutes at room temperature, the Tc-99m-N₂S₂-annexin V conjugate was purified by passage through a G-25 Sephadex (PD-10) column (available from Pharmacia) equilibrated with PBS. Fractions (1.0 ml) were collected, and those fractions containing annexin V were pooled. Protein concentration was determined by UV absorption at 280 nm. Tc-99m-annexin V (300 - 350 mg) conjugate solution was diluted and stored in PBS containing bovine serum albumin (BSA) at a final concentration of 15-20 mg BSA/ml PBS prior to injection.

### Example II

### procedure for Radiolabeling an Annexin - N₃S Chelate Conjugate

S-benzoylmercaptoacetylglycylglycylglycine (S-benzoyl MAG₃) is prepared in accordance with the procedures described in U.S. Patent No. 4,965,392. Then 25 micrograms of S-benzoylmercaptoacetylglycylglycylglycine is dissolved in 0.10 ml of 1.0 M carbonate buffer (pH 12). Then 75 mCi of Tc-99m pertechnetate is added in about 1.0 ml followed by 1.0 mg of freshly dissolved sodium dithionite (10 mg/ml). This mixture is heated at 100°C, plus or minus 4°C, for 3 minutes, then is cooled in an ice bath for 5 minutes to give Tc-99m-MAG₃ as determined by ITLC (CH₃CN solvent); anion exchange HPLC (Beckman AX, 10 micron 0.01M Na₂SO₄/0.01M Na₃PO₄, pH 7.0); and reverse phase HPLC (Beckman ODS, 5 micron 2% CH₃CN/0.01M Na₃PO₄, pH 7.0).

The Tc-99m-MAG₃ complex in carboxylate form is then esterified; 0.20 ml 1N HCl, 0.30 ml of 0.2M phosphate buffer pH 6.0, 10.0 mg 2,3,5,6-tetrafluorophenol (TFP) in 0.01 ml 90% CH₃CH, and 12.5 mg of EDC (1-(3-dimethylaminopropyl)-3-ethylcarbodiimide hydrochloride) in 0.10 ml of 90% CH₃CN are combined, and the reactants are mixed at room temperature (25°C, plus or minus 2°C) for 1 hour. At this point, a yield of Tc-99m-MAG₃-TFP ester as determined by ITLC (CH₃CH solvent); anion exchange HPLC (Beckman AX, 10 micron 0.01M Na₂SO₄/0.01M Na₃PO₄, pH 7.0); and reverse phase HPLC (Beckman ODS, 5 micron 34% CH₃CN/0.01M Na₃PO₄, pH 7.0). The preparation is purified using a C-18 Baker column. The reaction mixture is loaded in the column, washed two times with water and then eight times with 10% C₂H₅OH/0.01M Na₃PO₄, pH 7.0. The product is eluted with CH₃CN, and the solvent is removed prior to conjugation with annexin V.

The conjugation of the active ester to annexin V is carried out by adding annexin V in a phosphate buffer, pH 9.5, to the Tc-99m-MAG₃-TFP ester. The reaction is carried out for at least 30 minutes, and the desired radiolabeled annexin product is obtained by passage through a PD-10 gel filtration column.

### Example III

### Thrombus Imaging With a Radiolabeled Annexin

A. Animal Preparation - LA Vascular Thrombi. Fasting 25-30 kg Yorkshire swine were sedated with intramuscular Telazol (5-10 mg/kg) (commercially available from AVECO Co.) and commercially available Atropine (1 mg, Elkins-Sinn, Inc., Cherry Hill, HJ). Surital (200 mg) (commercially available from Abbott Laboratories) anaesthesia was administered intravenously. The animals were intubated and given inhalation anaesthesia of 1.5-2% halothane (commercially available from Abbott Laboratories) and O₂ sufficient to obtain a deep level of anesthesia and physiologic arterial blood gases. Continuous electrocardiographic monitoring was instituted using alligator clip electrodes. A cut down in the neck region was conducted, and an 8 french catheter (USCI Co, Billerica, MA) was placed in the right common carotid artery for blood pressure and arterial blood gas monitoring as well as for blood sampling.

The swine were placed in a right lateral decubitus position, and a lateral thoracotomy was performed to expose the heart. The incision was held open by a thoracotomy retractor. The pericardium was opened, and the left atrial appendage was isolated from the left atrium by a vascular cross- clamp. Rubber tipped forceps were used to gently crush the appendage. Five minutes later, ricinoleate (1 mg, ICN Pharmaceuticals, Costa Mesa, CA) and thrombin (50 mg, Johnson and Johnson Co., Arlington, Texas) were injected into the LAA (left atrial appendage) using a 27 Ga needle. The cross-clamp was removed 10 minutes later.

One hour after the crush injury, Tc-99m-annexin V, prepared in accordance with Example I above, was administered as an intravenous bolus dose in an ear vein. The intravenous line was then flushed with saline. In 7 animals, I-125 labeled ovalbumin was administered as a non-specific control preparable, for example, by the procedure described by Fraker et al., Biochem. Biophys. Res. Commun. 80: 849-57, 1978). Briefly, I-125-radiolabeled ovalbumin was prepared by the Iodogen method, employing 600 µg ovalbumin (Sigma Chemical Co., St. Louis, Missouri) and NaI-125 (2mCi, 0.92 nmol).

Image acquisition was performed as described below. At the end of the experimental procedure (generally about 150 minutes), the animals were sacrificed by an intravenous bolus dose of 80 mEq of KCl while the animals were still under general anaesthetic. A final blood sample was taken for well counting. The heart was rapidly excised, washed free of blood and dissected into samples for well counting. Additional samples of carotid artery, lung, liver, spleen, muscle and kidney were obtained in some animals.

B. Controls. Five different types of controls were used: open chest sham; closed chest sham; ovalbumin; indium platelets; and non-specific Tc-99m-labeled antibody.
1. Open Chest Sham. In three animals, the heart was exposed as above, but the left atrium was not isolated, crushed or injected with ricinoleate/thrombin. Marker images with a cobalt marker were performed as described below, and the Tc-99m-annexin V was injected 30-60 minutes after LAA exposure. Imaging and sample acquisition were identical to that described in A above.
2. Closed Chest Sham. In seven animals, an ear intravenous line was established. No thoracotomy was performed. Sedation and anesthesia were identical to that described in A above. The Tc-99m-annexin V (+/other control radionuclides, such as I-125 ovalbumin or In-111-platelets) was administered and image acquisition was performed.
3. Ovalbumin. I-125 ovalbumin was administered in 7 animals as a negative control protein. ovalbumin has a molecular size similar to that of annexin, and exhibits a slightly slower blood clearance.
4. Indium Platelets. In-111 platelet labeling was performed in 7 animals as a positive well counting label. In-111 radiolabeled platelets were prepared in accordance with the procedure described by Stratton et al., Am. J. Cardiol. 47: 874, 1981 and Stratton et al., Circulation 69: 561, 1984. Imaging was not attempted because of the long serum half-life of the In-111-platelets.
5. Non-specific Tc-Labeled Antibody. In a single experiment, a left atrial (LA) thrombi was created by the above method, but Tc-99m-Annexin V was not administered. Instead, a Tc-99m-Fab fragment of an antibody designated as HR-LU-10 was administered. NR-LU-10 is a 150 kilodalton molecular weight IgG2b monoclonal antibody that recognizes an approximately 40 kilodalton glycoprotein antigen expressed on most carcinomas. NR-LU-10 is a well characterized pancarcinoma antibody that has been safely administered to over 565 patients in human clinical trials. NR-LU-10-Fab is prepared in accordance with known techniques and is radiolabeled in accordance with the procedures described in J. Nucl. Med. 32: 1445-51, 1991 and by the modified C-18 Baker purified Tc-99m-N₂S₂-TFP procedure described in Example I for preparing radiolabeled annexin V. This Tc-99m-Fab conjugate was designed as a negative control for both well counting and imaging.

C. Imaging. A cobalt marker was placed on the exposed surface of the LAA and held in place with surgical tape affixed to the thoracotomy retractor. The tape was adjusted so that the marker generally moved with the LAA with each cardiac cycle. Marker images were acquired for 10 seconds in each planar view and 10 seconds in each tomographic slice. The cobalt marker was then removed.

A General Electric Starport camera with a general purpose collimator was used to acquire the Tc-99m images. Five minute planar acquisitions were performed sequentially in the left lateral, 45 LAO, and anterior views. These were followed by a 10 minute tomographic acquisition. This full set of 3 planar and 1 tomographic acquisitions was repeated for a total of 5 sets. Care was taken not to move the pig or imaging gantry during the entire imaging sequence.

Images were recorded on a Microdelta computer slaved to a VAX mainframe system. Images were stored on tape or on the VAX hard drive. Planar image analysis consisted of first viewing the image with the marker and recording the marker position on the viewing terminal screen. The first image acquired after Tc-99m-annexin V injection was used to define the cardiac blood pool. Each subsequent image was viewed and analyzed using the marker and initial blood pool as references. Each image was scored as positive, equivocal or negative.

Thirteen animals had left atrial thrombi created and were imaged as described above. Twelve animals had Tc-99m-annexin V injected for imaging, and one animal had a non-specific Tc-99m Fab injected as a control. Closed chest imaging was performed in 7 animals without atrial injury, and open chest sham experiments were performed in 3 animals. In animals with atrial thrombi, all planar images taken at less than 35 minutes after administration of Tc-99m-annexin V were negative. Nine of the atrial thrombi planar images taken at greater than 70 minutes were positive, 1 was equivocal and 2 were negative. All of the tomographic images of atrial thrombi were either positive (n=10) or equivocal (n=2) at imaging times of greater than 2 hours post injection. The average time in which the planar images became positive following administration of Tc-99m-annexin V was 82 minutes (35-135 minutes).

None of the closed chest control animals had positive images. One of the three open chest shams had a positive image at 85 minutes. This false positive is believed to result from the production of surgically-induced thrombi.

These results indicate that intravenous administration of Tc-99m-annexin V permitted acquisition of diagnostic images identifying atrial vascular thrombi within a short time period following conjugate administration.

D. Sample Collection. Samples (both blood and tissue), as described above, were weighed and placed in vials for immediate counting of Tc-99m. After the Tc-99m had decayed (typically at 5-7 days), the samples were re-assayed to obtain the I-125 counts. Each vial was counted for 1 minute. The counts were corrected for decay, then for weight, and recorded as counts per minute per gram. The counts per minute per gram for each sample were then normalized by dividing the counts per minute per gram of the final blood specimen. Consequently, the results for each sample were calculated in a ratio with the last blood sample, permitting meaningful comparison between animals. This procedure was performed for all radionuclides in a given experiment.

Well counting ratios were obtained for a variety of tissue samples. For the injured tissues and thrombi, multiple specimens were usually taken from the same animal. In those cases, the maximum ratio for any one specimen is reported, as well as the average of all specimens taken. The maximum for each animal was averaged across all animals and is reported as the Maximum Anx-V Ratio.

These results indicate that Tc-99m-annexin V localizes preferentially to atrial thrombi and injured left atrium, with the highest non-target localization occurring in the kidney. The kidney level is at least partially indicative of excretion of Tc-99m-annexin V via the renal route.

### Example IV

### Method for producing a cell expression clone of Annexin V

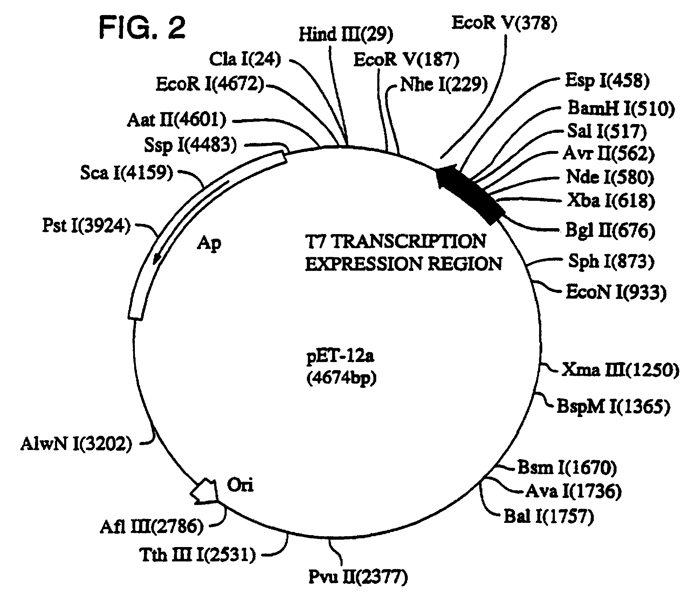

A parent clone, λHPAP1.6, is described in Funakoshi et al., "Primary Structure of Human Placental Anticoagulant Protein", *Biochemistry*, Vol. 26, pp. 8087-8092 (1987). Polymerase chain reaction (PCR) was used to amplify the annexin gene from the λHPAP1.6 parent clone. The sense primer (CAT ATG GCA CAG CTT CTC A) contained an NdeI restriction site (underlined) and the first 16 nucleotides of the annexin leader sequence, beginning with the ATG start codon. The antisense primer (GGA TCC **TTA** GTC ATC TTC TCC ACA) encoded the end of the coding sequence, a stop codon (bold) and BamHI restriction site (underlined). The PCR product and plasmid pET-12a (Figure 2) obtained from Novagen (Palo Alto, California) were each digested with NdeI and BamHI and ligated together with T4 DNA ligase. A portion of the ligation solution was transformed into an *E. coli* host strain and selected on nutrient agar plates containing ampicillin. Plasmid from the resultant clone was designated pET-12a-PAP1-E287G, 7/16/93, clone 1. Dideoxy DNA sequence analysis showed that this plasmid contained DNA that matched the wild-type annexin sequence except for two mutations [855 G → A (silent); 860 A - G (converts Glu-287 to Gly-287)].

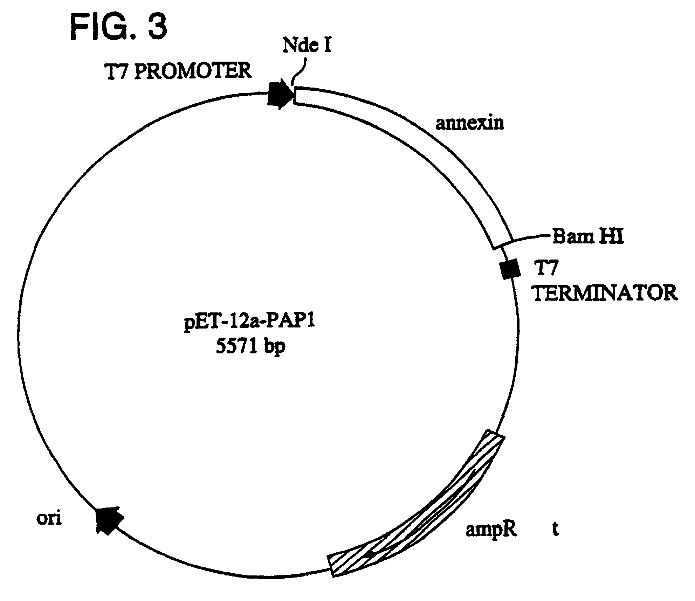

The sequence changes present in pET-12a-PAP-E287G, 7/16/93, clone 1 were corrected in the following manner. The plasmid was digested with restriction enzymes Sful and BamHI which excised a fragment of approximately 240 base pairs containing both mutations. The fragment was replaced with a 240 base pair fragment from an independent clone known to contain the wild-type sequence. The DNAs were ligated and transformed into an *E. coli* host. *E. coli* colonies containing plasmid DNA were selected on ampicillin containing nutrient agar plates. The resulting clone harbored plasmid pET-12a-PAP1, 3/7/94, clone 1 (Figure 3). DNA sequencing confirmed that the annexin coding sequence on the plasmid matched the wild-type sequence exactly.

The host strain BL21(DE3), received from Novagen, was transformed with the plasmid pET-12a-PAP1, 3/7/94, clone 1. A glycerol stock was made of the resulting transformant BL21(DE3) (pET-12a-PAP1, 3/7/94, clone 1) and stored at ≤-65°C.

Growth of *E. coli* BL21(DE3) (pET-12a-PAP1, 3/7/94, clone 1) in liquid culture at 37°C resulted in accumulation of the Annexin V protein in the *E. coli* periplasmic space.

### Example V

### Procedure for modification of Annexin V

The plasmid pET-12a is described above in Example IV. Annexin amino acid variant genes were placed between the NdeI and BamHI restriction sites on the pET-12a plasmid.

An independent modification of annexin V was created by site-directed amino acid alteration, utilizing PCR amplification. To accomplish this alteration, oligonucleotides which anneal in the antisense direction of the 3' end of annexin were created. The particular alteration of annexin V, described herein, is a replacement of a cysteine residue (position 316) with alanine. In order to achieve this alteration, the nucleotide sequence was changed from TGT to GCA. The oligonucleotide includes a BamHI restriction enzyme site and a stop codon.

The sense oligonucleotide anneals within the T7 promoter region of pET-12a, just upstream from the NdeI restriction site or the 5' end of annexin V. The following are the anti-sense and sense oligonucleotide sequence, respectively:
(Nx168 Cys to Ala antisense) 5' GTACCTGGATCCTTAGTCATCTTCTCCGCGAGCAGCAGAAGAGCTTTCTT 3'; and (T7 sense)

5' CGAAATTAATACGACTCACTATAGGG 3'.

The Nx168 and T7 oligonucleotides were synthesized using DNA synthesizer, model 381A (Applied Biosystem Inc., model 381A). After synthesis was complete both oligonucleotides were deprotected as per appendix 5 of the above manufacturer's protocol. Purification was done using a Sephadex™ G-25 column (Pharmacia, Uppsala, Sweden).

PCR reactions were done using UlTma™ polymerase (Perkin Elmer, Norwalk, CT). The reactions were performed using approximately 10ng of pET12a-annexin V template, 30 pMol of T7 and Nx168 oligonucleotides, and 0.4 mM of each nucleotide. Mineral oil overlay was included. The reactions were placed in a Coy temperature cycler model 110P and incubated for five minutes at 94°C. Approximately 2 minutes into the incubation, 0.5 units of polymerase was added. The reactions were then cycled 30 times at 94°C for 30 seconds, 55°C for one minute, and 74°C for one minute. The reactions were then incubated for 5 minutes at 74°C, and then soaked at 15°C.

The resulting annexin-ala substituted gene was then purified using a DNA purification kit (Promega Magic™ PCR Preps DNA Purification System, Madison, WI) according to manufacture's protocol.

Annexin-ala and pET-12a were then digested with NdeI and BamHI (Gibco/BRL, Gaithersburg, MD) restriction enzymes. Digested products were then purified by agarose gel electrophoresis. Appropriate bands were excised and purified using GeneClean II® Kit (Bio 101, Inc., La Jolla, CA) according to manufacture's protocol. The annexin-ala and pET-12a were then ligated with T4 DNA ligase (Promega) for 12-18 hours at 4°C.

Annexin-ala-pET-12a were transformed into the *E. coli* strain DH5α Max. efficiency competent cells (Gibco/BRL). Any other suitable host strain for the pET-12a plasmid may also be used. The *E. coli* transformed cells are placed on LB agar (Gibco/BRL), supplemented with 100 ug/mL ampicillin (International Biotechnologies Inc., New Haven, CT) and incubated for 12-18 hours at 37°C. A suitable colony, evident to one of ordinary skill in the art, was then selected for placement in Terrific Broth (Gibco/BRL), which has been supplemented with 100 ug/mL ampicillin (International Biotechnologies Inc.)

Several colonies from the LB agar plates are selected and expanded. The plasmid DNA is purified using Promega's Wizard miniprep purification kit. Once plasmid DNA is purified, colonies are screened by sequence analysis in order to confirm that a particular clone selected contains the amino acid modification or alteration.

## Claims

1. An annexin - containing conjugate suitable for radiolabeling with a diagnostic imaging agent comprising:
an annexin; and
an N₂S₂ or N₃S compound capable of complexing a radionuclide,
wherein the compound is covalently bound to the annexin.

2. The annexin - containing conjugate of Claim 1 wherein the annexin is annexin V.

3. The annexin - containing conjugate of Claim 1 wherein the annexin is modified and the modification comprises the deletion of a cysteine amino acid.

4. The annexin - containing conjugate of Claim 1 wherein the annexin is modified and the modification comprises the replacement of a cysteine amino acid with a non - sulphur containing amino acid.

5. The annexin - containing conjugate of Claim 4 wherein the non - sulphur containing amino acid comprises alanine.

6. The annexin - containing conjugate of Claim 1 of the following formula:

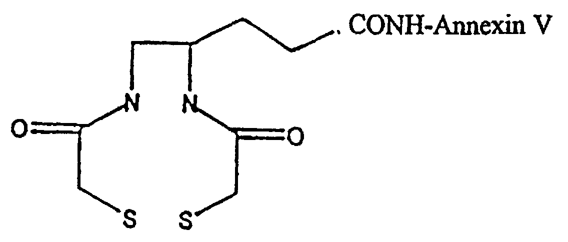

7. A radiolabeled annexin for imaging vascular thrombi, which radiolabeled annexin comprises:
an annexin;
an N₂S₂ or N₃S compound capable of complexing a radionuclide,
wherein the compound is covalently bound to the annexin;
a diagnostic radionuclide complexed by the compound.

8. The radiolabeled annexin of Claim 7 wherein the annexin is annexin V.

9. The radiolabeled annexin of Claim 7 wherein the annexin is modified and the modification comprises the deletion of a cysteine amino acid.

10. The radolabeled annexin of Claim 7 wherein the annexin is modified and the modification comprises replacement of a cysteine amino acid with a non - sulphur containing amino acid.

11. A radiolabeled annexin of Claim 10 wherein the non - sulphur containing amino acid comprises alanine.

12. The radiolabeled annexin of Claim 7 of the following formula:

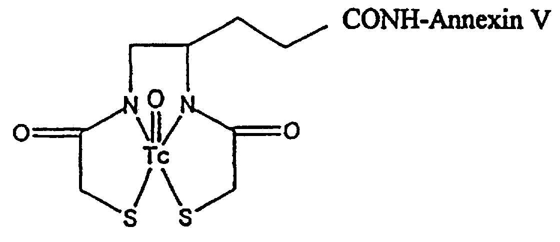

13. The radiolabeled annexin of Claim 7, wherein the radionuclide is selected from the group consisting of Tc - 99m, Re - 186, Re - 188, Pd - 100, Bi - 212, Pb - 212, Pd - 109 and Cu - 67.

14. The radiolabeled annexin of Claim 7, wherein the radionuclide is Tc - 99m.

## Patentansprüche

1. Annexin-enthaltendes Konjugat, das geeignet ist für ein radioaktives Markieren mit einem diagnostischen bildgebenden Agens, welches umfaßt:
ein Annexin; und
eine N₂S₂- oder N₃S-Verbindung, welche in der Lage ist, ein Radionuklid zu komplexieren, wobei die Verbindung kovalent an das Annexin angebunden ist.

2. Annexin-enthaltendes Konjugat nach Anspruch 1, bei welchem das Annexin Annexin V ist.

3. Annexin-enthaltendes Konjugat nach Anspruch 1, bei welchem das Annexin modifiziert ist und die Modifikation die Streichung einer Cystein-Aminosäure umfaßt.

4. Annexin-enthaltendes Konjugat nach Anspruch 1, bei welchem das Annexin modifiziert ist und die Modifikation den Ersatz einer Cystein-Aminosäure mit einer Aminosäure, welche keinen Schwefel enthält, umfaßt.

5. Annexin-enthaltendes Konjugat nach Anspruch 4, bei welchem die Aminosäure, welche keinen Schwefel enthält, Alanin umfaßt.

6. Annexin-enthaltendes Konjugat nach Anspruch 1 der folgenden Formel:

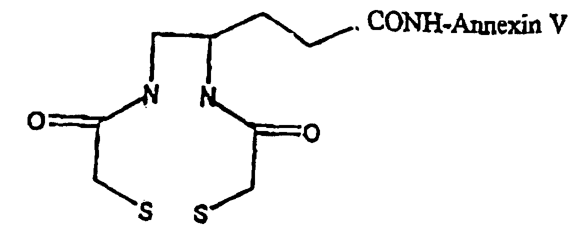

7. Radioaktiv markiertes Annexin zum Bildgeben vaskulärer Thrombi, welches radioaktiv markiertes Annexin umfaßt:
ein Annexin;
eine N₂S₂- oder N₃S-Verbindung, welche in der Lage ist, ein Radionuklid zu komplexieren, wobei die Verbindung kovalent an das Annexin angebunden ist;
ein diagnostisches Radionuklid, das durch die Verbindung komplexiert ist.

8. Radioaktiv markiertes Annexin nach Anspruch 7, bei welchem das Annexin Annexin V ist.

9. Radioaktiv markiertes Annexin nach Anspruch 7, bei welchem das Annexin modifiziert ist und die Modifikation die Streichung einer Cystein-Aminosäure umfaßt.

10. Radioaktiv markiertes Annexin nach Anspruch 7, bei welchem das Annexin modifiziert ist und die Modifikation einen Ersatz einer Cystein-Aminosäure mit einer Aminosäure, welche keinen Schwefel enthält, umfaßt.

11. Radioaktiv markiertes Annexin nach Anspruch 10, bei welchem die Aminosäure, welche keinen Schwefel enthält, Alanin umfaßt.

12. Radioaktiv markiertes Annexin nach Anspruch 7 der folgenden Formel:

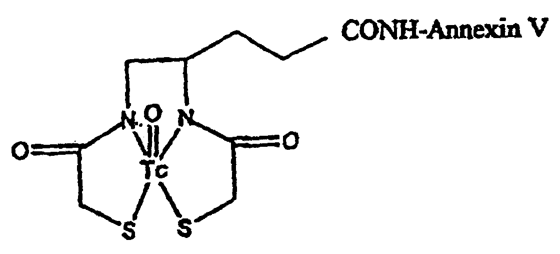

13. Radioaktiv markiertes Annexin nach Anspruch 7, bei welchem das Radionuklid ausgewählt ist aus der Gruppe bestehend aus Tc-99m, Re - 186, Re - 188, Pd - 100, Bi - 212, Pb - 212, Pd - 109 und Cu - 67.

14. Radioaktiv markiertes Annexin nach Anspruch 7, bei welchem das Radionuklid Tc - 99m ist.

## Revendications

1. Conjugué contenant une annexine, apte au marquage radio-actif avec un agent d'imagerie de diagnostic, comprenant :
une annexine ; et
un composé de formule N₂S₂ ou N₃S apte à la complexation d'un radionucléide,
dans lequel le composé est lié par covalence à l'annexine.

2. Conjugué contenant une annexine suivant la revendication 1, dans lequel l'annexine est l'annexine V.

3. Conjugué contenant une annexine suivant la revendication 1, dans lequel l'annexine est modifiée et la modification comprend la délétion d'un amino-acide cystéine.

4. Conjugué contenant une annexine suivant la revendication 1, dans lequel l'annexine est modifiée et la modification comprend le remplacement d'un amino-acide cystéine par un amine-acide ne contenant pas de soufre.

5. Conjugué contenant une annexine suivant la revendication 4, dans lequel l'amino-acide ne contenant pas de soufre comprend l'alanine.

6. Conjugué contenant une annexine suivant la revendication 1, répondant à la formule suivante :

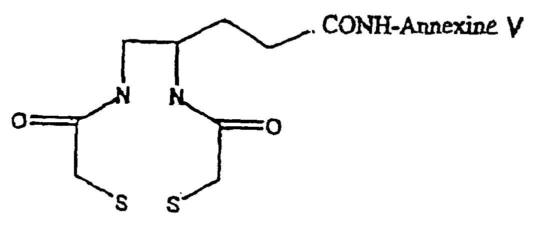

7. Annexine radiomarquée pour l'imagerie de thrombus vasculaires, annexine radiomarquée qui comprend :
une annexine ;
un composé de formule N₂S₂ ou N₃S apte à la complexation d'un radionucléide,
le composé étant lié par covalence à l'annexine ;
un radionucléide de diagnostic complexé par le composé.

8. Annexine radiomarquée suivant la revendication 7, dans laquelle l'annexine est l'annexine V.

9. Annexine radiomarquée suivant la revendication 7, dans laquelle l'annexine est modifiée et la modification comprend la délétion d'un amino-acide cystéine.

10. Annexine radiomarquée suivant la revendication 7, dans laquelle l'annexine est modifiée et la modification comprend le remplacement d'un amino-acide cystéine par un amino-acide ne contenant pas de soufre.

11. Annexine radiomarquée suivant la revendication 10, dans laquelle l'amino-acide ne contenant pas de soufre comprend l'alanine.

12. Annexine radiomarquée suivant la revendication 7, répondant à la formule suivante :

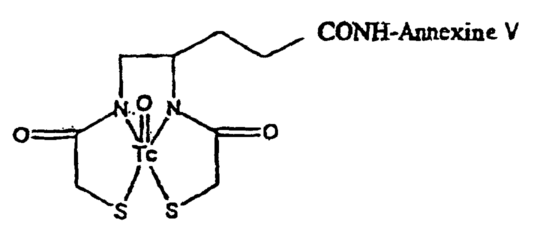

13. Annexine radiomarquée suivant la revendication 7, dans laquelle le radionucléide est choisi dans le groupe consistant en Tc-99m, Re-186, Re-188, Pd-100, Bi-212, Pb-212, Pd-109 et Cu-67.

14. Annexine radiomarquée suivant la revendication 7, dans laquelle le radionucléide consiste en Tc-99m.
